# FASCICULE DE BREVET EUROPEEN

(11) **EP 2 166 113 B1**
(45) Date de publication et mention de la délivrance du brevet: **07.05.2014**
(21) Numéro de dépôt: 09305851.9
(22) Date de dépôt: 15.09.2009
(51) Int. Cl.: C12Q 1/68

(54) **Procédé de détection, d'identification et de quantification de Brachyspira et kit pour la mise en oeuvre d'un tel procédé**
Verfahren zur Erkennung, Identifizierung und Quantifizierung von Brachyspira, und Kit zur Umsetzung eines solchen Verfahrens
Method for detecting, identifying and quantifying Brachyspira and kit for implementing such a method

(30) Priorité: 22.09.2008 FR 0805191
(43) Date de publication de la demande: 24.03.2010
(73) Titulaire: Centre Européen d'Expertise et de Recherche sur les Agents Microbiens CEERAM, 44240 La Chapelle sur Erdre (FR); RC Services SAS, 85500 Les Herbiers (FR)
(72) Inventeur: Lebeau, Benoît, 44700 Orvault (FR); Loisy-Hamon, Fabienne, 44700 Orvault (FR); Fourrier, Angélique, 44000 Nantes (FR); Guillaume, Jean-Michel, 56460 Serent (FR)
(74) Mandataire: Laget, Jean-Loup

(56) Documents cités:
- ATYEO R F ET AL: "Differentiation of Serpulina species by NADH oxidase gene (nox) sequence comparisons and nox-based polymerase chain reaction tests" VETERINARY MICROBIOLOGY, vol. 67, no. 1, 1 juin 1999 (1999-06-01), pages 47-60, XP002520476 ISSN: 0378-1135
- DAVIS A J ET AL: "The Brachyspira hyodysenteriae ftnA gene: DNA vaccination and real-time PCR quantification of bacteria in a mouse model of disease" CURRENT MICROBIOLOGY, NEW YORK, NY, US, vol. 50, no. 6, 1 juin 2005 (2005-06-01), pages 285-291, XP002409195 ISSN: 0343-8651
- ROHDE JUDITH ET AL: "Differentiation of porcine Brachyspira species by a novel nox PCR-based restriction fragment length polymorphism analysis." JOURNAL OF CLINICAL MICROBIOLOGY JUL 2002, vol. 40, no. 7, juillet 2002 (2002-07), pages 2598-2600, XP002520477 ISSN: 0095-1137
- PHILLIPS NYREE D ET AL: "Development of a two-step nested duplex PCR assay for the rapid detection of Brachyspira pilosicoli and Brachyspira intermedia in chicken faeces" VETERINARY MICROBIOLOGY, vol. 116, no. 1-3, août 2006 (2006-08), pages 239-245, XP002520478 ISSN: 0378-1135
- LA T ET AL: "Development of a multiplex-PCR for rapid detection of the enteric pathogens Lawsonia intracellularis, Brachyspira hyodysenteriae, and Brachyspira pilosicoli in porcine faeces" LETTERS IN APPLIED MICROBIOLOGY, vol. 42, no. 3, mars 2006 (2006-03), pages 284-288, XP002520479 ISSN: 0266-8254
- CALDERARO A ET AL: "Comparative evaluation of molecular assays for the identification of intestinal spirochaetes from diseased pigs" VETERINARY MICROBIOLOGY, vol. 118, no. 1-2, novembre 2006 (2006-11) , pages 91-100, XP002520480 ISSN: 0378-1135
- WEISSENBOECK HERBERT ET AL: "Amplification and sequencing of Brachyspira spp. specific portions of nox using paraffin-embedded tissue samples from clinical colitis in Austrian pigs shows frequent solitary presence of Brachyspira murdochii" VETERINARY MICROBIOLOGY, vol. 111, no. 1-2, novembre 2005 (2005-11) , pages 67-75, XP002520481 ISSN: 0378-1135
- CASTELAIN SANDRINE ET AL: "TaqMan amplification system with an internal positive control for HCV RNA quantitation." JOURNAL OF CLINICAL VIROLOGY : THE OFFICIAL PUBLICATION OF THE PAN AMERICAN SOCIETY FOR CLINICAL VIROLOGY NOV 2004, vol. 31, no. 3, novembre 2004 (2004-11), pages 227-234, XP002520482 ISSN: 1386-6532
- STANTON THADDEUS B ET AL: "Collateral effects of antibiotics: Carbadox and metronidazole induce VSH-1 and facilitate gene transfer among Brachyspira hyodysenterae strains" APPLIED AND ENVIRONMENTAL MICROBIOLOGY, vol. 74, no. 10, mai 2008 (2008-05), pages 2950-2956, XP002559756 ISSN: 0099-2240
- MALINEN E ET AL: "Comparison of real-time PCR with SYBR green I or 5'-nuclease assays and dot-blot hybridization with rDNA-targeted oligonucleotide probes in quantification of selected faecal bacteria" MICROBIOLOGY, SOCIETY FOR GENERAL MICROBIOLOGY, READING, GB, vol. 149, no. 1, 1 janvier 2003 (2003-01-01), pages 269-277, XP002451760 ISSN: 1350-0872

### SEQUENCE LISTING

<110> CENTRE EUROPEN D'EXPERTISE ET DE RECHERCHE SUR DES AGENTS MICROBIENS - CEERAM RC SERVICES SAS
<120> Procédé de détection et de quantification de Brachyspira et kit pour la mise en oeuvre d'un tel procédé
<130> C08-0570FR
<160> 12
<170> PatentIn version 3.3
<210> 1
   <211> 24
   <212> DNA
   <213> Brachyspira hyodysenteriae
<220>
   <221> forward primer
   <222> (1)..(24)
<400> 1
   atgtcccata taaacatcga tgcc 24
<210> 2
   <211> 25
   <212> DNA
   <213> Brachyspira hyodysenteriae
<220>
   <221> reverse primer
   <222> (1)..(25)
<400> 2
   gttggtggcg tagttaaaga tccta 25
<210> 3
   <211> 21
   <212> DNA
   <213> Brachyspira hyodysenteriae
<220>
   <221> probe
   <222> (1)..21)
<400> 3
   cacctctcaa actttcagga c 21
<210> 4
   <211> 31.
   <212> DNA
   <23.3> Brachyspira pilosicoli
<220>
   <221> forward primer
   <222> (1)..(31
<400> 4
   cactagaaca gtctcctata gcataaacat t 31
<210> 5
   <211> 28
   <212> DNA
   <213> Brachyspira pilosicoli
<220>
   <221> reverse rimer
   <222> (1)..(28)
<400> 5 agattattta gagactttac ctaatggt 28
<210> 6
   <211> 20
   <212> DNA
   <213> Brachyspira pilosicoli
<220>
   <221> probe
   <222> (1)..(20)
<400> 6
   ctcatcttta gaagatttca 20
<210> 7
   <211> 31
   <212> DNA
   <213> Brachyspira intermedia
<220>
   <221> forward primer
   <222> (1)..(31)
<400> 7
   gtatagcaat agagaatgca tgtatagctt c 31
<210> 8
   <211> 32
   <212> DNA
   <213> Brachyspira intermedia
<220>
   <221> reverse primer
   <222> (1)..(32)
g400> 8
   gta aaaatag tttatgaaga agatactggacg 32
<210> 9
   <211> 20
   <212> DNA
   <213> Brachyspira intermedia
<220>
   <221> probe
   <222> (1)..(20)
<400> 9
   catggttgtt tttagaagca 20
<210> 10
   <211> 1247
   <212> DNA
   <213> Brachyspira intermedia
<220>
   <221> gene
   <222> (1)..(1247)
<220>
   <221> misc...feature
   <222> (869)..(869)
   <223> n is a, c, g, or t
<400> 10
   atgctggtac atgggcagta aaaactttga aagctacaga tcctaattgt caggtagtta 60
   cttatgatag aaacgataat atatctttcc tagcttgcgg tatcgcactt tgggttggag 120
   gagtagttaa agatcctaaa ggtttatttt atgctagtcc tgaaagctta aaaagcgaag 180
   gcgtagaagt tcatatggga catgatgtta ctaaaataga ctgggctaac aaaaaattat 240
   atgtaaaaga actaaaaaca ggaaaagagt ttgaagacac ttatgacaaa cttattcttg 300
   ctactggttc ttggcctgta actcctccta tagaaggatt aaaacaggaa ggaactactt 360
   atggtcttaa aaaaggtatt ttcttctcta aactttatca gcaaggtcaa gaaattatcg 420
   aagaattaaa aaaacctgaa gtaaaaaaag ttatggtagt tggtgctggt tatataggtg 480
   ttgaacttat agaagcattc aaaaatcatg gtaaagaagt tatattgatg gaagctttgc 540
   ctagagttat gggtaattac ttcgataaag aaatcactga tgaagctgaa aaaagaatca 600
   aagaagctgg catagaaatg catttaggtg aaactgttaa gaaatttgaa ggtgatgaca 660
   gagttaaaaa agttgttact gacaaaggtt cttatgaagt agatatggta gttatgtctg 720
   ttggtttcaa acctaatagc gacctttata aagactattt agaaactctt cctaatggtg 780
   ctattgtagt agatactact atgaaaacta gcaaagatcc tgatgtttat gctataggtg 840
   actgtgctag cgtatattca agagcttcng aaaagcaaga atatattgct ttagctacta 900
   atgctgtaag aatgggtatt gttgctgcta acaatgcttt aggcaaacat gttgaatact 960
   gcggaactca aggttctaat gctatatgcg tatttggata taatatggct tctactggtt 1020
   ggtctgaaga gactgctaag aaaaaaggat taaaagcaaa atctaatttc ttcaaagatt 1080
   ctgaaagacc agaatttatg cctactcatg aagaagtatt agtaaaaata gtttatgaag 1140
   aagatactgg acgtttatta ggtgctcaaa ttgcttctaa aaacaaccat gctgaagcta 1200
   tacatgcatt ctctattgct atacaaagag gtatgactgt tcaggac 1247
<210> 11
   <211> 1268
   <212> DNA
   <213> Brachyspira pilosicoli
<220>
   <221> gene
   <222> (1)..(1268)
<220>
   <221> misc_feature
   <222> (107)..(107)
   <223> n is a, c, g, or t
<220>
   <221> misc_feature
   <222> (170)..(170)
   <223> n is a, c, g, or t
<220>
   <221> misc_feature
   <222> (200)..(200)
   <223> n is a, c, g, or t
<220>
   <221> misc_feature
   <222> (206)..(206)
   <223> n is a, c, g, or t
<220>
   <221> misc_feature
   <222> (212)..(212)
   <223> n is a, c, g, or t
<220>
   <221> misc_feature
   <222> (287)..(287)
   <223> n is a, c, g, or t
<220>
   <221> misc_feature
   <222> (420)..(420)
   <223> n is a, c, g, or t
<220>
   <221> misc_feature
   <222> (497)..(497)
   <223> n is a, c, g, or t
<220>
   <221> misc_feature
   <222> (641)..(641)
   <223> n is a, c, g, or t
<400> 11
   atgccggcac atgggctgca aaaactttaa aagctacaga tcctaattgt caggtagtta      60
   cttatgatag aaacgataat atatctttct tagcttgcgg tattgcnctt tgggttggcg     120
   gcgtagtaaa agatcctaaa ggtttattct atgctagtcc tgaaggtttn aaaagcgaag     180
   gaatagaagt ttatatgggn catgangtaa cnaaaataga ttgggctaac aaaaaaatga     240
   ctgttaaaga attaaaaact ggtaaagagt ttgaagataa ttatganaaa cttattcttg     300
   ctactggttc ttggcctgta actcctccta ttgagggctt aaaacaagaa ggtactactt     360
   atggtcttaa aaaaggtatt ttcttctcta aactttatca acaaggtcaa gatattattn     420
   atgaaatagc taaaccagaa gtaaaaagag ttatggtagt tggtgctggt tatataggtg     480
   ttgaacttat agaagcnttc aaaaatcatg gtaaagaagt tatattaatg gaagctatgc 540
   ctagagttat ggctaactat tttgacaaag agattactga tgaggctgaa aaaagaatta 600
   aagatgctgg tatagaattg catttaggtg aaactgttaa naaatttgaa ggtgatgaca 660
   gagttaaaaa agttgttact gacaaaggtt cttatgatgt agatatggta gttatgtctg 720
   ttggtttcag acctaatagc gaactttata aagattattt agagacttta cctaatggtg 780
   ctattgtagt tgatactact atgaaatctt ctaaagatga gaatgtttat gctataggag 840
   actgttctag tgtatattct tgttcttcta aatctcatga atatattgct ttagctacaa 900
   atgctgtaag aatgggtatt gttgctgcta ataatatttt aggtaaaaaa gttaattatt 960
   gcggtacaca aggttctaac gctatttgtg tatttggtta taatatggct tctactggtt 1020
   ggtctgaaga gactgctaag aagaaaggtt taaaagttaa atctaacttc tttagagatg 1080
   ctgaaagacc tgagtttatg cctacttatg aagatgtatt agtaaaaatc gtttatgaag 1140
   aaggtactgg aagattatta ggtgctcaga ttgcttctaa acataatcat gctgaagcta 1200
   tacacgcatt ctctcttgct atagctaatg aaatgactgt tcaagatttt gcattatctg 1260
   atttcttc 1268
<210> 12
   <211> 1380
   <212> DNA
   <213> Brachyspira hyodysenteriae
<220>
   <221> gene
   <222> (1)..(1380)
<400> 12
   ttttaataag gggttaaatt attatgaaag ttattgtaat aggttgtaac catgctggta 60
   catgggcagc aaaaactttg aaagctacag atcctaattg tcaagtagtt acttacgata 120
   gaaatgataa tatatctttc ttagcctgcg gtatcgcact ttgggttggt ggcgtagtta 180
   aagatcctaa aggattattc tatgctagtc ctgaaagttt gagaggtgaa ggcatcgatg 240
   tttatatggg acatgatgtt actaaaatag actgggctaa caaaaaatta tgtgtaaaag 300
   aactaaaaac aggaaaagag tttgaagaca cttacgataa acttattctt gctactggtt 360
   cttggcctgt aactcctcct atcgaaggct taaaacaaga aggaactact tacggactta 420
   aaaaaggtat tttcttctct aagctttatc agcaaggaca agaaattatt gatgaaatag 480
   ctaaaccaga tgttaaaaaa gttatggtag ttggtgctgg atacataggt gttgaactta 540
   tagaagcatt caaaaaccat ggtaaagaag ttatcttaat ggaagctatg cctagagtta 600
   tggctaacta ctttgataaa gaaatcactg atgaagctga aaaaagaatc aaagaagctg 660
   gcatagaaat gcatttaggt gaaactgtta agaaatttga aggtgatgac agagttaaaa 720
   aagttgttac tgacaaaggt tcttatgatg tagatatggt agttatgtct gttggtttca 780
   gacctaataa tgaactttat aaagattatt tagaaacttt acctaatggt gctattgtag 840
   tagatactac tatgaaaact actaaagatc ctgatgtatt tgctataggt gactgtgcta 900
   ctgtatattc aagagcttct gaaaaacaag aatatattgc tttagctact aatgctgtaa 960
   gaatgggtat tgttgctgct aataatgctt taggaaaaca tgttgaatat tgcggtactc 1020
   aaggttctaa tgctatttgt gtatttggat acaatatggc ttctactggt tggtctgaag 1080
   aaactgctaa gaaaaaagga ttaaaagtaa aatctaactt cttcaaagat tctgaaagac 1140
   cagaatttat gcctactaat gaagatgttt tagtaaaaat catttatgaa gaaggcagca 1200
   gacgtttatt aggtgctcaa atagcttcta aacacaatca tgctgaagct attcatgcat 1260
   tctctcttgc tatacaaaat ggtatgactg ttgatcaatt tgcattgtca gatttcttct 1320
   tcctacctca ctacaacaaa ccattatctt ggatgactat ggttgcttat actgctaaat 1380

## Description

La présente invention se rapporte à un procédé de détection, d'identification et de quantification par amplification par PCR en temps réel pour le diagnostic rapide de *Brachyspira hyodysenteriae, Brachyspira pilosicoli* et *Brachyspira intermedia* présent dans l'environnement ou sur des mammifères et des oiseaux dans le cadre d'une application en diagnostic de routine.

La présente invention se rapporte également à un kit de détection mis en oeuvre par le procédé susmentionné.

### Cas du porc :

Ces bactéries du genre *Brachyspira* sont à l'origine de diverses affections digestives chez le porc. De plus la réglementation des facteurs de croissance dans l'alimentation porcine a comme principale conséquence la recrudescence des pathologies digestives liées aux *Brachyspira.* Différents groupes correspondant aux cinq principales espèces chez le porc ont été décrits: *B. hyodysenteriae, B. intermedia, B. murdochii, B. innocens, B. pilosicoli.* Les affections liées à ces différentes espèces n'ont pas la même incidence économique. *B. hyodysenteriae* est responsable de la dysenterie porcine caractérisée par une diarrhée muccohémorragique. Cette pathologie entraîne des pertes économiques importantes du fait d'un retard de croissance des animaux infectés. C'est une des pathologies digestives les plus coûteuses pour l'industrie porcine. *B.pilosicoli* est l'agent responsable de la spirochétose intestinale porcine, les animaux infectés présentent une diarrhée non hémorragique, et une réduction des indices de croissance. L'incidence de pathologie des autres espèces de *Brachyspira* isolées chez le porc est mal élucidée. *B.intermedia* est fréquemment isolée de fèces de porc en engraissement présentant des symptômes diarrhéiques.

*Brachyspira hyodysenteriae* est l'agent de la dysenterie porcine appelée entérite hémorragique, maladie identifiée dans tous les pays ou l'élevage porcin est développé et affectant principalement les porcs en engraissement. La dysenterie porcine est une maladie dont les répercussions économiques sont importantes du fait de la mortalité et, surtout du coût des traitements et des retards de croissance engendrés.

Le principal mode de contamination des élevages résulte de l'introduction d'animaux infectés. Les animaux guéris représentent un danger important car ils peuvent excréter le micro-organisme après les symptômes au-delà de 70 jours. D'autres modes de contamination peuvent être rencontrés (contamination par des effluents, contamination par des animaux porteurs, nourriture contaminée, objet souillé...). La période d'incubation varie de 5 à 37 jours avec une moyenne de 11 jours. Durant cette période d'incubation, les animaux peuvent déjà être excréteurs car ils éliminent la bactérie entre 3 et 18 jours après un contact infectant. Dans les cas typiques, l'état général des animaux est d'abord peu modifié, mais ils se nourrissent sans enthousiasme. Progressivement, les porcs maigrissent et peuvent devenir cachectiques. La majorité des animaux guérit en une ou deux semaines, mais l'indice de consommation augmente et le GMQ (Gain Moyen Quotidien) peut diminuer au-delà de 6 semaines. Dans les formes les plus graves, les animaux sont très affaiblis, ils se déshydratent et meurent en environ 5 jours.

*Brachyspira pilosicoli* est responsable d'une infection expérimentalement reproductible et connue sous les noms de spirochétose intestinale du porc. Des infections ont été décrites dans la plupart des pays où l'élevage porcin est développé. Les facteurs prédisposants sont mal connus et invoquent la composition de l'aliment, le procédé de granulation, l'utilisation des facteurs de croissance. Le mode de contamination semble voisin de celui observé pour les infections *Brachyspira hyodysenteriae.* Les animaux les plus sensibles sont les porcs en engraissement. Expérimentalement, la période d'incubation a une durée de 5 à 16 jours, parfois plus et, dans les conditions naturelles, elle est de l'ordre de 7 jours. Les premiers signes cliniques sont une légère détérioration de l'état général et l'émission de fèces ramollies. L'état général des animaux est altéré, l'infection s'accompagne de fièvre, d'un faible indice de consommation et d'un retard de croissance important. Des cas de mortalité sont éventuellement observés (2% des animaux), mais l'importance de la maladie est avant tout liée aux pertes économiques résultants de la diminution de la croissance et du coût des traitements.

*Brachyspira intermedia* est isolée de porcs, souffrant éventuellement de diarrhées mais, à l'exception d'une étude, la maladie n'a pas été reproduite. Le pouvoir pathogène pour le porc est encore controversé, même si, après le sevrage, quelques cas de diarrhée sont associés à la présence de cette espèce dans l'intestin.

### Cas de la volaille :

Plus connues chez le porc pour les diarrhées et le retard de croissance qu'elles occasionnent, les *Brachyspira* peuvent aussi affecter les volailles.

Chez la volaille, la maladie dénommée spirochétose intestinale aviaire, se manifeste par des fèces caecales liquides, par une baisse de la ponte (5 à 15%) et un retard de croissance. Les *Brachyspira* tapissent les parois caecales et perturbent la digestion. Ces bactéries sont résistantes dans le milieu extérieur et les volailles se contamineraient surtout par l'ingestion de fèces caecales.

Par exemple ces bactéries sont responsables des chutes de ponte inexpliquées :
- l'infection expérimentale de poules *par Brachyspira pilosicoli* provoque une forte baisse de la production d'oeufs (- 22% par rapport au témoin)
- l'infection *par Brachyspira intermedia* diminue la production de 11%.

Classiquement, il était admis que *Brachyspira hyodysenteriae* avait un spectre d'hôtes étroit, limité au porc, même si cette bactérie est capable d'infecter de manière transitoire et sans symptôme d'autres espèces animales en contact avec des fèces de porcs infectés.

En 2004, Jansson et al. rapportent l'isolement de sept souches *de Brachyspira hyodysenteriae* à partir des fèces ou du cloaque de canards. Cinq souches provenaient d'animaux d'élevage et deux d'animaux sauvages.

### Diagnostic effectué selon l'état de la technique

Le diagnostic des infections liées à *Brachyspira* est principalement basé sur l'isolement du germe en culture et sa caractérisation biochimique. Ils sont isolés à partir de fèces ou de contenu intestinal. L'isolement nécessite, après un pré-enrichissement sur bouillon, l'utilisation de milieu sélectif tel que le milieu Blood Agar Media contenant des antibiotiques (spectinomycine, rifampicine). Cette technique permet d'obtenir des résultats en 72 heures.

De plus il est nécessaire de réaliser des analyses microbiologiques complémentaires pour le diagnostic de l'espèce (hémolysine, pouvoir indologène, productions de diverses enzymes...). Cette identification reste longue et fastidieuse.

Les techniques de diagnostic indirect par mise en évidence d'anticorps (ELISA, hémagglutination indirecte, micro-agglutination...) sont parfois utilisées pour détecter des élevages infectés, mais elles ne permettent ni un diagnostic individuel ni la détection des animaux porteurs.

Des tests PCR classiques, en point final, permettant l'identification des *Brachyspira* ont été décrits dans la littérature et comparés aux méthodes traditionnelles. Pronost et al. (Revue de Médecine Vétérinaire, Vol. 150, p. 803 à 808, 1999 - "Apports de la PCR pour l'identification des souches de Brachyspira pathogènes chez le porc*")* ont montré l'intérêt de la PCR en tant qu'outil de caractérisation d'un isolat de *Brachyspira* obtenu en culture pure. Il s'agit d'une amplification des gènes codant pour l'ARNr 16S, l'ARNr 23S, la NADH oxydase ou l'hémolysine. Ces tests sont généralement effectués sur des cultures car il est difficile d'extraire l'ADN directement à partir de fèces.

Des diagnostics définitifs par PCR couplé à la méthode culture ne peuvent alors être établis que dans un délai de 5 à 7 jours.

Toutefois, La et al. (J. of Chimical Microbiology, Vol. 41, p. 3372-3375, 2003- Development of a duplex PCR assay for detection of Brachyspira hyodysenteriae and Brachyspira pilosicoli in pig feces) ont décrit un test de PCR en point final, utilisable sur l'ADN extrait des fèces. Ce test amplifie une portion du gène codant pour l'ARNr 16S pour *Brachyspira pilosicoli* et la NADH oxydase de *Brachyspira hyodysenteriae.* Il permet d'identifier plus rapidement les animaux infectés mais manque de sensibilité et de reproductibilité.

La publication de R.F Atyeo «*differentiation of Serpulina species by NADH oxidase gene (nox) sequence comparisons and nox-based polymerase chain reaction tests*» décrit un procédé de détection plus ou moins efficace (NOX1 à NOX4) du gène *nox* de l'espèce *Serpulina* et de l'espèce *Brachyspira aalborgi* utilisant la PCR en point final. Un séquençage et un alignement des séquences résultantes de ces espèces ont été analysés afin de définir un arbre phylogénétique. Ce document enseigne ainsi que les tests NOX1 et NOX4 ont générés des produits à partir de toutes les souches étudiées, tandis que NOX2 et NOX3, non. En particulier, NOX3 ne permet pas, malgré des modifications, d'amplifier la souche ATCC 43994 de *Brachyspira aalborgi.*

La publication de Antony J. Davis « the Brachyspira hyodysenteriae ftnA Gene : DNA Vaccination and Real-Time PCR Quantification of Bacteria in a mouse Model of desease » décrit l'utilisation de la PCR en temps réel pour déterminer le nombre de B. *hyodysenteriae* au sein du *caeca* de souris infectés. Dans ce document, il ne s'agit pas ici de quantifier le gène *nox,* mais le gène *mcpA* (page 286, 2^{ème} colonne, 1^{er} paragraphe).

La publication de Judith Rohde « Differentiation of Porcine Brachyspira species by a novel nox PCR-based restriction fragment length polymorphism analysis » a pour objet de trouver un test permettant d'identifier par des tests de routine PCR suivi d'une RFLP (polymorphisme de longueur de fragments de restriction) toutes les espèces *Brachyspira.* La PCR utilisé cible le gène *nox.* Pour cela une amorce sens 5'-TAG C(CT)T GCG GTA T(CT)G C(AT)C TTT GG-3' et une amorce anti-sens 5'-CTT CAG ACC A(CT)C CAG TAG AAG CC-3' ont été mis au point.

La publication de Nyree D.Phillips « Development of a two-setp nested PCR assay for the rapid detection of *Brachyspira pilocicoli* and *Brachyspira intermedia* in chiken faeces » décrit un test afin de détecter et d'identifier Brachyspira pilosicoli et Brachyspira intermedia en faisant, lors d'une première étape, une PCR duplex sur les gènes *nox* et 16S ARN de fèces de poulet lavées et lors d'une deuxième étape, une PCR duplex nichée qui amplifie le gène 16SrARN de *Brachyspira pilocicoli* et le gène *nox* de *Brachyspira intermedia* à partir des amplicons produits lors de la première étape.

La publication de A.Caldero et al. « Comparative evaluation of molecular assays for the idientification of intestinal spirochaetes from diseased pigs » décrit des comparatifs entre diffétentes méthodes de l'art antérieurs pour détecter l'espèce *Brachyspira.*

La plupart de ces documents décrivent ainsi des méthodes de détection de l'espèce Brachyspira se basant sur la technique de la PCR du gène *nox* ou autres ou de la combinaison de la PCR et de la RFLP (polymorphisme de longueur de fragments de restriction) ou encore par PCR duplex ou PCR duplex niché. Par conséquent, il n'était pas évident pour un homme du métier de trouver la solution revendiquée par la présente demanderesse et ce même si celui-ci avait connaissance de la technique de la PCR en temps réel. Il n'est pas évident de passer d'une technique à une autre.

En conclusion, il reste encore un besoin dans le développement de procédé permettant un diagnostic rapide, efficace, **très sensible et reproductible** de *Brachyspira hyodysenteriae, Brachyspira pilosicoli* et *Brachyspira intermedia* dans le cadre d'une application en diagnostic de routine.

Un but de la présente invention est de fournir un nouveau procédé de détection, d'identification et de quantification de *Brachyspira hyodosenteriae, Brachspira pilosicoli* et *Brachyspira intermedia* à partir d'un échantillon biologique prélevé dans l'environnement, chez les mammifères et/ou les oiseaux.

Un autre but de la présente invention est de fournir des kits (ou trousses) pour la mise en oeuvre du procédé susmentionné.

A cet effet, l'invention concerne un procédé de détection, d'identification et de quantification du gène *nox* (gène NADH oxidase) de l'espèce *Brachyspira* issu d'un échantillon environnemental ou animal tel que des fèces de mammifères et en particulier de porc et/ou de volaille, par réaction de polymérisation en chaîne (PCR), caractérisé en ce que la réaction de polymérisation en chaîne (PCR) étant en temps réel, le procédé comprend les étapes consistant à :
(i) analyser ledit échantillon susceptible de contenir des *Brachyspira* par PCR avec un milieu réactionnel comprenant une amorce sens apte à se lier spécifiquement à la région 5'-3' du gène *nox,* une amorce anti-sens apte à se lier spécifiquement à la région 3'-5' du gène *nox,* et une sonde d'hybridation moléculaire marquée fluorescente détectable qui se lie spécifiquement à l'amplicon résultant de la réaction PCR,
(ii) mesurer et quantifier, par l'intermédiaire d'un système optique d'un thermocycleur ou d'un système équivalent, la présence d'émission de fluorescence indiquant, de façon proportionnelle, la quantité d'amplicon à chaque cycle PCR, de manière à pouvoir détecter la présence de *Brachyspira* à partir dudit échantillon environnemental ou animal,
ledit procédé étant caractérisé en ce que :
- les amorces de *Brachyspira hyodysenteriae* comprennent les séquences suivantes : ATG TCC CAT ATA AAC ATC GAT GCC (amorce sens) et GTT GGT GGC GTA GTT AAA GAT CCT A (amorce anti-sens), la sonde de *Brachyspira hyodysenteriae* comprend la séquence suivante : CAC CTC TCA AAC TTT CAG GAC ;
- les amorces de *Brachyspira pilosicoli* comprennent les séquences suivantes : CAC TAG AAC AGT CTC CTA TAG CAT AAA CAT T (amorce sens) et A GAT TAT TTA GAG ACT TTA CCT AAT GGT (amorce anti-sens), la sonde de *Brachyspira pilosicoli* comprend la séquence suivante : CTCATCTTTAGAAGATTTCA ; et
- les amorces de *Brachyspira intermedia* comprennent les séquences suivantes : GTA TAG CAA TAG AGA ATG CAT GTA TAG CTT C (amorce sens) et GTA AAA ATA GTT TA T GAA GAA GAT ACT GGA CG (amorce anti-sens), la sonde de *Brachyspira intermedia* comprend la séquence suivante : CATGGTTGTTTTTAGAAGCA.

Le genre *Brachyspira* appartient à l'ordre des Spirochaetales et à la famille des « Brachyspiraceae ». Ce sont des bactéries hélicoïdales, Gram négatif, mobiles et anaérobiques. Les espèces d'intérêt vétérinaire se cultivent à des températures comprises entre 37°C et 42°C dans des bouillons trypticase soja ou coeur-cervelle contenant 10 p. cent de sérum (sérum foetal de veau, de boeuf ou mouton). Les souches de *Bracchyspira hyodysenteriae* sont fortement hémolytiques alors que celles de *Brachyspira pilosicoli* et *intermedia* sont faiblement hémolytiques.

Les inventeurs ont découvert l'intérêt du gène NADH oxidase *(nox)* dans la différenciation des espèces de *Brachyspira, par PCR en temps réel.*

La NADH oxydase *(nox)* réduit l'oxygène moléculaire en eau. Cette réduction permet à la bactérie de vivre à proximité d'un tissu qui utilise de l'oxygène, comme l'intestin. Le gène *nox* est une cible intéressante en PCR, en effet celui-ci est conservé au sein des espèces de *Brachyspira*, il est au minimum identique à 86,3% au sein des espèces de *Brachyspira.*

À partir de l'alignement de toutes les espèces, les inventeurs ont pu observer des régions conservées spécifiques des trois bactéries cibles permettant de trouver le couple amorces/sonde approprié pour chacune d'elles. Pour *Brachyspira pilosicoli,* la région intéressante se situe entre 421 paires de bases et 521 paires de bases (figure 1). Il y a une région de 100 nucléotides spécifiques pour cette espèce. Pour *Brachyspira hyodysenteriae,* la région intéressante se situe entre 1128 paires de bases et 1216 paires de bases (figure 3). Il y a une région de 88 nucléotides spécifiques pour cette espèce. Pour *Brachyspira intermedia,* la région intéressante se situe entre 48 paires de bases et 150 paires de bases (figure 2). Il y a une région de 102 nucléotides spécifiques pour cette espèce.

Dans ces régions spécifiques des couples d'amorces/sonde ont été déterminés pour chacune des espèces pour une analyse par PCR en temps réel.

La PCR en temps réel se distingue de la PCR classique par le fait que l'analyse de la réaction se fait dans la phase exponentielle, plus reproductible, et non en point final. A la différence d'une PCR classique, la PCR en temps réel utilise une sonde fluorescente, spécifique de la cible, qui permet la quantification et la caractérisation en temps réel de l'amplicon synthétisé. C'est le couple amorces/sonde qui induit la grande spécificité de la PCR en temps réel.

La technologie de PCR en temps réel est en effet basée sur la détection et la quantification d'un « rapporteur » fluorescent. L'augmentation du signal fluorescent est directement proportionnelle à la quantité d'amplicons générés durant la réaction de PCR. En observant la quantité de fluorescence émise à chaque cycle, il devient possible de suivre la réaction PCR durant sa phase exponentielle où l'augmentation significative de la quantité d'amplicons est en corrélation directe avec la quantité initiale de matrice cible. Le processus peut être automatisé du début à la fin rendant cette technologie très performante pour des applications d'analyse à grande échelle.

Pour visualiser la réaction de PCR en temps réel, deux possibilités se présentent :
- l'utilisation d'un agent intercalant comme le « Sybr Green® » qui se fixe sur l'ADN double brin;
- ou l'utilisation d'une sonde permettant d'accroître la spécificité de l'amplification (sondes TaqMan®, sondes TaqMan® MGB^{™}...).

Ainsi, dans un premier mode de réalisation d'un procédé qui ne fait pas partie de l'invention, la sonde marquée détectable du procédé de détection et de quantification du gène *nox* de l'espèce Brachyspira est un agent intercalant apte à se lier à l'ADN double brin de l'amplicon résultant de la réaction PCR, tel que Sybr Green^{®}.

Dans un mode de réalisation, la sonde marquée détectable est une sonde moléculaire apte à accroître la spécificité de l'amplification comme les sondes TaqMan^{®}, TaqMan^{®} MGB^{™}.

Parmi les différentes sondes existantes, les sondes TaqMan sont utilisées de préférence. C'est un oligonucléotide d'une longueur de 20-30 bases marqué avec un fluorophore (rapporteur) à l'extrémité 5' et un inhibiteur de fluorescence (désactiveur) à l'extrémité 3'. Durant la PCR, en présence d'une cible, la sonde s'hybride spécifiquement sur l'ADN cible. Le « désactiveur » inhibe alors l'émission de fluorescence du « rapporteur ». Pendant la réaction, la *Taq* polymérase possédant une activité exonucléase 5'-3', dégrade la sonde et libère le rapporteur permettant l'émission de fluorescence. Un accroissement de la fluorescence est donc observé au fur et à mesure de l'amplification lorsque la région recherchée est présente.

Les sondes peuvent être composées de « rapporteur » et « désactiveur » différents qui se distinguent chacun par une absorbance spécifique. Ainsi le Fam est détecté à 530 nm, le Tamra à 582 nm.

Au milieu réactionnel du procédé de détection, d'identification et de quantification du gène *nox* de l'espèce *Brachyspira,* une molécule fluorescente (ROX) est ajoutée de préférence, elle correspond à la référence passive car elle ne participe pas à la réaction d'amplification PCR. Elle permet de normaliser le signal complexe obtenu lors de la mesure de fluorescence.

L'intensité du signal de fluorescence est proportionnelle au nombre de copies d'ADN synthétisées à chaque cycle de PCR. L'évolution de l'amplification est représentée par une courbe sigmoïdale où l'abscisse correspond au nombre de cycles et l'ordonnée à l'amplitude du signal généré appelée ΔRn. Le Rn (pour Normalized Rapporteur) est le rapport de l'intensité de fluorescence émise par le rapporteur sur celle émise par la référence passive. La référence passive permet d'éliminer le bruit de fond de fluorescence.

Dans les exemples ci-dessous de la présente invention, des sondes Applied Biosystems TaqMan® MGB^{™} qui comprennent un rapporteur coloré en 5' et un « désactiveur » non-fluorescent (NFQ) en extrémité 3' ont été utilisées. Le NFQ possède l'avantage de réduire le bruit de fond, ce qui permet d'obtenir une meilleure précision de la quantification. La partie MGB (Minor Groove Binding) stabilise la sonde hybridée et augmente la température de fusion (Tf). En se fixant à l'extrémité 3' d'une sonde, le MGB se replie dans le petit sillon formé par l'ADN double brin pour stabiliser l'hybridation. L'effet de cette stabilisation est une augmentation de la température de fusion et la partie MGB produit un effet de « régulation de la Tf » quand le pourcentage d'Adénosine-Thymine augmente. Cette technologie permet d'obtenir des sondes plus courtes et spécifiques pour une Tf donnée (68 - 70°C).

Des sondes TaqMan® MGB^{™} ont donc été définies pour respecter les paramètres principaux et obtenir la meilleure sensibilité.

L'invention a également pour objet un kit (ou trousse) de détection ou de quantification du gène *nox* de l'espèce *Brachyspira* pour la mise en oeuvre du procédé d'identification et de quantification tel que décrit ci-dessus, comprenant des réactifs appropriés pour l'amplification du gène *nox* de l'espèce *Brachyspira* issu d'un échantillon environnemental ou animal tel que des fèces de mammifères et en particulier de porc et/ou de volaille.

En particulier, le kit (ou trousse) de détection ou de quantification du gène *nox* de l'espèce *Brachyspira* pour la mise en oeuvre du procédé d'identification et de quantification tel que décrit ci-dessus, comprend un milieu réactionnel comportant :
(i) une amorce sens apte à se lier spécifiquement à la région 5'-3' du gène *nox*,,
(ii) une amorce anti-sens apte à se lier spécifiquement à la région 3'-5' du gène *nox,*
(iii) une sonde d'hybridation moléculaire marquée fluorescente détectable qui se lie spécifiquement à l'amplicon (gène *nox*) résultant de la réaction PCR,
(iv) éventuellement, une molécule fluorescente ROX est ajoutée au mélange réactionnel, de manière à normaliser le signal complexe obtenu lors de la mesure de fluorescence,
(v) des dNTPs et des polymérases présents en large excès dans le milieu réactionnel.

Le kit est caractérisé en ce que :
- les amorces de *Brachyspira hyodysenteriae* comprennent les séquences suivantes : ATG TCC CAT ATA AAC ATC GAT GCC (amorce sens) et GTT GGT GGC GTA GTT AAA GAT CCT A (amorce anti-sens), la sonde de *Brachyspira hyodysenteriae* comprend la séquence suivante : CACCTCTCAAACTTTCAGGAC;
- les amorces de *Brachyspira pilosicoli* comprennent les séquences suivantes : CAC TAG AAC AGT CTC CTA TAG CAT AAA CAT T (amorce sens) et A GAT TAT TTA GAG ACT TTA CCT AAT GGT (amorce anti-sens), la sonde de *Brachyspira pilosicoli* comprend la séquence suivante : CTCATCTTTAGAAGATTTCA ; et
- les amorces de *Brachyspira intermedia* comprennent les séquences suivantes : G TAT AGC AAT AGA GAA TGC ATG TAT AGC TTC (amorce sens) et GTA AAA ATA GTT TA T GAA GAA GAT ACT GGA CG (amorce anti-sens), la sonde de *Brachyspira intermedia* comprend la séquence suivante : CATGGTTGTTTTTAGAAGCA.

Pour valider le résultat et s'affranchir de faux positifs ou de faux négatifs qui peuvent être dus, par exemple, à un artefact lié aux réactifs utilisés ou à une mauvaise purification des acides nucléiques, le kit peut comprendre également :
(vi) un témoin interne positif (IPC),
(vii) un témoin positif
(viii) un témoin négatif.

Selon un mode de réalisation d'un kit qui ne fait pas partie de l'invention, la sonde marquée détectable est un agent intercalant apte à se lier à l'ADN double brin de l'amplicon résultant de la réaction PCR, tel que Sybr Green^{®}.

Selon un mode de réalisation, la sonde marquée détectable est une sonde moléculaire apte à accroître la spécificité de l'amplification comme les sondes TaqMan^{®}, TaqMan^{®} MGB^{™}.

L'invention sera mieux comprise et d'autres buts, détails, caractéristiques et avantages de celle-ci apparaîtront plus clairement à la lecture de la description suivante d'exemples de réalisation, en référence aux figures suivantes :
- la figure 1 présente la région d'intérêt du gène *nox* pour *Brachyspira pilosicoli* ;
- la figure 2 présente la région d'intérêt du gène *nox* pour *Brachyspira intermedia* ;
- la figure 3 présente la région d'intérêt du gène *nox* pour *Brachyspira hyodysenteriae* ;
- la figure 4 présente les valeurs de Ct correspondant à l'amplification résultant d'une PCR temps réel sur ADN extrait d'une souche de référence, dilution 10⁻⁵ à 10⁻⁸ pour *B. pilosicoli*, 10⁻⁴ à 10-⁷ pour *B. hyodysenteriae* et 10⁻² à 10⁻⁵ pour *B. intermedia* ;
- la figure 5 présente les valeurs de Ct correspondant à l'amplification résultant d'une PCR temps réel FAST sur ADN extrait de souche de référence, dilution 10⁻⁴ à 10⁻⁷ pour *B. pilosicoli* ;
- la figure 6 montre les résultats obtenus avec des échantillons suspectés de spirochétose de selles de porc avec les souches de références *B. pilosicoli* et *B.hyodysenteriae pour des valeurs Ct ("Threshold cycle"- cycle seuil) obtenues avec des acides nucléiques, pur ou dilué au 1*/*10, ainsi que des valeurs de Ct de l'IPC (contrôler interne d'amplification)* ;
- et la figure 7 présente les valeurs de Ct correspondant à l'amplification des témoins positifs à *Brachyspira pilosicoli, Brachyspira hyodosenteriae et Brachyspira intermedia.*

### Exemple 1 : Sensibilité, spécificité, robustesse et optimisation de la PCR Brachyspira

### A - Echantillons

### 1 - Les cultures bactériennes

Les souches types des différentes espèces de *Brachyspira* proviennent de l'American Type Culture Collection (ATCC) et de la collection de l'institut Pasteur (CIP). Les autres souches de *Brachyspira* ou leur ADN ainsi que les différentes espèces bactériennes utilisées pour la mise en oeuvre du test de PCR en temps réel ont été fournies par différents laboratoires partenaires.

**Tableau 1 : Souches de référence des Brachyspira spp.**

| **Espèces bactériennes** | **Origine** |
|---|---|
| *B. hyodysenteriae* | ATCC 27164 |
| *B. pilosicoli* | ATCC 51339 |
| *B. intermedia* | CIP 105833 |
| *B. alvinipulli* | CIP 105681 |
| *B. murclochii* | CIP 105832 |

### 2 - Les prélèvements terrain

Sur la base de prélèvements terrain réalisés en élevage sur des animaux qui ont présenté des signes cliniques de spirochétose, la recherche de *Brachyspira* a été également réalisée.

### 3 - Milieux et conditions de culture

Les souches de références de *Brachyspira* ont été mises en culture sur bouillon Brain Heart Infusion (BHI) contenant 10 % de sang défibriné de cheval. Les cultures ont été incubées à 37°C en anaérobiose pendant 2 à 5 jours en fonction des souches.

### B - Extraction des acides nucléiques

### 1 - Les cultures bactériennes

L'ADN a été extrait par lyse thermique par la méthode PrepMan® Ultra d'Applied Biosystems. Pour chaque souche 1 mL de culture a été centrifugé 3 minutes à 16 000 g ; le culot bactérien a été repris dans 200 µL de tampon PrepMan® Ultra et placé 10 minutes à 100°C dans un bain-marie sec. L'échantillon a été ensuite centrifugé 3 minutes à 16 000 g puis le surnageant a été transféré dans un nouveau micro tube. Les lysats bactériens ainsi obtenus ont été conservés à - 20°C jusqu'à amplification.

### 2 - Les fèces

Cette extraction a été réalisée sur 2 g d'échantillon de fèces. La concentration bactérienne a été réalisée par ajout de 10 mL de PBS stérile. La contamination artificielle a été faite par ajout de 1 mL de la culture bactérienne de référence. Le tube a été brièvement agité puis centrifugé 10 minutes à 5 000g. Le culot a été conservé.

### Echantillons à analyser suite à des prélèvements par des vétérinaires

L'extraction a été réalisée sur 100 µL de contenu intestinal. La concentration bactérienne a été obtenue par ajout de 900 µL d'eau stérile puis le tube a été brièvement agité et centrifugé 10 min à 5 000g. Le culot a été conservé.

Ensuite, l'extraction des acides nucléiques a été réalisée en utilisant la plateforme NucliSens® miniMAG^{™} avec les réactifs NucliSens® Magnetic Extraction Reagents de chez bioMérieux selon les recommandations du fournisseur.

### C - PCR en Temps réel Brachyspira

### 1 - Alignements des séquences

Pour réaliser un alignement des différents génomes de *Brachyspira* (*intermedia, pilosicoli et hyodysenteriae*), il a été nécessaire de trouver les séquences nucléotidiques correspondantes dans la banque de données Genbank. Un alignement a été ensuite réalisé à l'aide du logiciel CLC Free Workbench. Ainsi, il a été possible de localiser les régions les plus conservées intéressantes pour définir les amorces et les sondes des différentes espèces de *Brachyspira.*

### 2 - Définition des amorces et des sondes

Après avoir déterminé une région intéressante, la séquence consensus résultant de l'alignement a été analysée dans le logiciel Primer express 3.0 d'Applied Biosystems ou le logiciel Primer 3 plus. Ces logiciels permettent de rechercher le couple amorces/sonde le plus approprié pour la PCR en temps réel permettant d'obtenir une PCR efficace.

*Des couples d'amorces*/*sonde ont été déterminés pour chacune des espèces pour les régions spécifiques codant le gène nox (tableau 2).*

**Tableau 2 : couples amorces/sondes**

| **Couple *B.hyodysenteriae*** | **Séquences** |
|---|---|
| Amorce F | ATGTCCCATATAAACATCGATGCC |
| Amorce R | GTTGGTGGCGTAGTTAAAGATCCTA |
| Sonde | CACCTCTCAAACTTTCAGGAC |
| | |

| **Couple *B.pilosicoli*** | **Séquences** |
|---|---|
| Amorce F | CACTAGAACAGTCTCCTATAGCATAAACATT |
| Amorce R | AGATTATTTAGAGACTTTACCTAATGGT |
| Sonde | CTCATCTTTAGAAGATTTCA |
| | |

| **Couple *B.intermedia*** | **Séquences** |
|---|---|
| Amorce F | GTATAGCAATAGAGAATGCATGTATAGCTTC |
| Amorce R | GTAAAAATAGTTTATGAAGAAGATACTGGACG |
| Sonde | CATGGTTGTTTTTAGAAGCA |

L'amplicon obtenu pour *Brachyspira pilosicoli* avec le couple *B. pilosicoli* a une taille de 153 pb. Pour *Brachyspira hyodysenteriae* avec le couple *B.hyodysenteriae* on obtient un amplicon de 89 pb et celui de *Brachyspira intermedia* avec le couple inter une taille de 103 pb.

### 3 - Kit PCR en temps réel

Pour cette étude, le TaqMan® Master Mix d'Applied Biosytems a été utilisé (Tableau 3). Des travaux précédents ont montré son efficacité en PCR en temps réel.

**Tableau 3 : Kit, Mélange réactionnel et Programme utilisé pour les PCR TaqMan Temps réel.**

| **Kit** | **Mélange Réactionnel pour 1 Tube** | **Programme** |
|---|---|---|
| TaqMan ® Universal PCR Master Mix Applied Biosystems | 6, 25 µL H20 | 2 minutes 50 °C |
| | 12,5 µL Master Mix | |
| | µL amorce Sens 10 µM | 10 minutes 95°C |
| | 0,5 µL amorce Antisens 10 µM | 15 secondes 95°C |
| | 0,25 µL Sonde 10 µM | 1 minute 60°C X 45 |
| | 5 µL d'échantillon | |

L'ensemble des PCR en temps réel ont été réalisées sur des appareils Step One ou 7300 SDS d'Applied Biosystems.

### 4 - Validation de la spécificité

La spécificité des amorces et sondes définies doit être testée sur d'autres organismes (bactéries, virus) que les *Brachyspira spp.* ciblés (Tableau 4). Les organismes testés sont responsables d'infections intestinales et possèdent le même habitat.

**Tableau 4: Liste des organismes testés pour valider la spécicité de la PCR en Temps réel.**

| | |
|---|---|
| **Virus** | - Rotavirus |
| | - Astrovirus |
| | - Adénovirus |
| | - Norovirus GI |
| | - Norovirus GII |
| | - Virus de l'Hépatite A |
| | - Virus de l'Hépatite E |
| **Bactéries** | - *Escherichia coli* |
| | - *Campylobacter coli* |
| | - *Campylobacter jejuni.* |
| **Bactéries** | - *Listeria spp.* |
| | - *Vibrio spp.* |
| | - *Salmonella spp.* |
| | - *Brachyspira suanatina* |
| | - *Brachyspira murdochii* |
| | - *Brachyspira alvinipulli* |

### 5 - Robustesse

Pour valider l'essai de PCR en temps réel développé, des tests de répétabilité intra-essai et de reproductibilité inter-essai et inter-manipulateur sont indispensables. Cinq réplicats par dilution ont ainsi été testé par PCR en temps réel pour chaque essai. Les dilutions limites ont été aussi intéressantes à observer car elles définissent la sensibilité du test, c'est-à-dire le nombre de copie minimum d'ADN bactérien détecté.

### 6 - Optimisation de la PCR en temps réel

### • Fast Universal PCR Master Kit

Afin d'assurer un gain de temps optimal dans l'obtention des résultats, le kit TaqMan® Fast Universal PCR Master Mix d'Applied Biosystems (Tableau 5) a également été testé.

**Tableau 5 : Kit, Mélange réactionnel et Programme utilisé pour les PCR TaqMan Temps réel Fast.**

| **Kit** | **Mélange Réactionnel pour 1 Tube** | **Programme** |
|---|---|---|
| TaqMan® Fast Universal PCR Master Mix d' Applied Biosystems | 6, 25 µL H20 | 20 secondes 95°C |
| | 12,5 µL Fast Master Mix | |
| | 0,5 µL amorce Sens 10 µM | 1 seconde 95°C |
| | 0,5 µL amorce Antisens 10 µM | 20 secondes 60°C X 45 |
| | 0,25 µL Sonde 10 µM | |
| | 5 µL d'échantillon | |

### • Contrôle interne d'amplification

Afin de contrôler chaque réaction, un contrôle interne d'amplification (ou IPC : Internal Positive Control) est rajouté dans le Master Mix de PCR en temps réel avec les amorces/sondes de chaque espèce de *Brachyspira* (tableau 6). L'IPC est marqué par un fluorophore VIC. L'utilisation de ce dernier permet de vérifier l'absence d'inhibiteurs et de vérifier que l'amplification PCR a fonctionné correctement.

**Tableau 6 : Kit, Mélange réactionnel et Programme utilisé pour les PCR TaqMan Temps réel avec contrôle interne.**

| **Kit** | **Mélange Réactionnel pour 1 Tube** | **Programme** | |
|---|---|---|---|
| TaqMan ® Universal PCR Master Mix d'Applied Biosystems | 3, 25 µL H20 | 2 minutes 50 °C | |
| | 12,5 µL Fast Master Mix | | |
| | 2,5 µL IPC Mix | 10 minutes 95°C | |
| | 0,5 µL IPC DNA | | |
| | 0,5 µL amorce Sens 10 µM | 15 secondes 95°C | |
| | 0,5 µL amorce Antisens 10 µM | | |
| | 0,25 µL Sonde 10 µM | 1 minute 60°C | X 45 |
| | 5 µL d'échantillon | | |

### Exemple 2 : Validation de la sensibilité et de la spécificité

Chaque couple d'amorce et sonde a été testé sur les 3 souches bactériennes (tableau 7) afin d'observer leur spécificité et leur sensibilité.

**Tableau 7 : Souches de référence des Brachyspira spp.**

| **Espèces bactériennes** | **Origine** |
|---|---|
| *B. hyodysenteriae* | ATCC 27164 |
| *B. pilosicoli* | ATCC 51339 |
| *B. intermedia* | CIP 105833 |

Des études antérieures ont démontré l'efficacité du kit TaqMan® Master Mix d'Applied Biosytems, par conséquent les essais en temps réel sont réalisés avec celui-ci. Les trois couples ont été testés avec les souches de référence extraite par lyse thermique à l'aide de la solution PrepMan® Ultra d'Applied Biosystems (figure 4).

Le tableau 8 met en évidence la sensibilité obtenue pour les trois couples. Une bonne efficacité d'amplification a été observée pour chacun des couples, ceci a son importance lorsqu'on cherche à détecter des bactéries en très faible quantité dans un échantillon. De plus, l'essai de PCR temps réel développé est sensible pour chaque souche de *Brachyspira* cible compte tenu des valeurs de Ct limites obtenus correspondant aux limites de détection de la méthode et estimés entre 1 et 5 copies de génome.

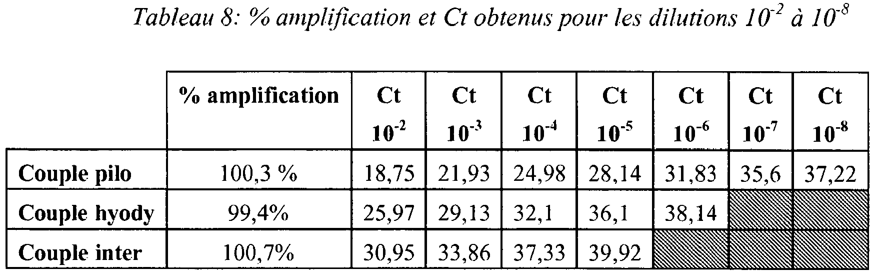

La spécificité de chaque couple a ensuite été testée sur divers organismes (bactéries et virus) pouvant se retrouver dans diverses infections digestives chez le porc et la volaille (tableau 4). Aucune amplification n'a été observée que ce soit avec les autres espèces de *Brachyspira* ou avec les 7 espèces bactériennes et les 5 virus testés. L'essai temps réel développé est également spécifique aux *Brachyspira.* De plus, aucune réaction croisée n'a été observée entre les 3 espèces ciblées. Les 3 tests de PCR en temps réel définis sont donc sensibles et spécifiques.

Dans le tableau 9 sont donnés les résultats de la répétabilité et de la reproductibilité. La répétabilité a été faite avec 5 réplicats par dilution et la reproductivité a été mesurée à partir de 2 essais réalisés dans les mêmes conditions avec les mêmes dilutions mais par des manipulateurs différents. L'efficacité de l'amplification est supérieure à 95% pour tous les essais et les variations de Ct ne sont pas significatives donc nous pouvons conclure que les 3 essais sont répétables et reproductibles.

**Tableau 9: Test de répétabilité intra manipulateur (intra essai et inter essai) et reproductibilité inter manipulateur par PCR temps réel sur dilution limites d'ADN extrait des souches de référence.**

| **Couple pilo** | Répétabilité intra-essai | | Répétabilité inter-essai | | Reproductibilité manipulateur | | Reproductibilité manipulateur | |
|---|---|---|---|---|---|---|---|---|
| Dilution | 10⁻⁶ | 10⁻⁷ | 10⁻⁶ | 10⁻⁷ | 10⁻⁶ | 10⁻⁷ | 10⁻⁶ | 10⁻⁷ |
| Variation Ct | 0,93 | 0,93 | 0,35 | 1,32 | 1,26 | 1,04 | 1,43 | 0,56 |
| | | | | | | | | |

| **Couple hyody** | Répétabilité intra-essai | | Répétabilité inter-essai | | Reproductibilité manipulateur | | Reproductibilité manipulateur | |
|---|---|---|---|---|---|---|---|---|
| Dilution | 10⁻⁵ | 10⁻⁶ | 10⁻⁵ | 10⁻⁶ | 10⁻⁵ | 10⁻⁶ | 10⁻⁵ | 10⁻⁶ |
| Variation Ct | 1,59 | 0,14 | 1,35 | 0,44 | 1,44 | 0,4 | 1,66 | 0,56 |
| | | | | | | | | |

| **Couple inter** | Répétabilité intra-essai | | Répétabilité inter-essai | | Reproductibilité manipulateur | | Reproductibilité manipulateur | |
|---|---|---|---|---|---|---|---|---|
| Dilution | 10⁻⁴ | 10⁻⁵ | 10⁻⁴ | 10⁻⁵ | 10⁻⁴ | 10⁻⁵ | 10⁻⁴ | 10⁻⁵ |
| Variation Ct | 0,84 | 0,51 | 0,82 | 1,40 | 0,76 | 0,33 | 1,09 | 1,9 |

De plus des essais ont été réalisés sur un autre type d'appareil en Temps réel (Stratagene MX3000). Les résultats obtenus sont similaires, par conséquent, le test de détection est applicable sur différents types d'appareil Temps réel sans perte de sensibilité.

Une des exigences des laboratoires vétérinaires est la rapidité du diagnostic. Dans cette optique, le kit TaqMan® Fast Universal PCR Master Mix d'Applied Biosystems a été testé sur les échantillons terrains. Celui-ci permet de réaliser une PCR en temps réel en 50 min, soit une réduction de moitié du temps de manipulation. Le kit a été validé au niveau de la sensibilité (figure 5), la reproductibilité et testé sur des échantillons de fèces.

Aucune différence significative n'a été observée par rapport à l'utilisation du kit TaqMan® Master Mix d'Applied Biosytems en terme de sensibilité, de spécificité et d'efficacité sur des échantillons de fèces. Par conséquent, le présent demandeur a réussi à développer un test répondant à l'une des attentes des laboratoires vétérinaires afin de pouvoir intervenir dans les élevages de porc dans des délais très courts.

Le développement de cet outil de détection repose sur le fait de réaliser la PCR en temps réel directement à partir des fèces afin de gagner du temps dans le diagnostic. Or les fèces sont des matrices difficiles à extraire et pouvant contenir de nombreux inhibiteurs. Pour vérifier que l'amplification se déroule parfaitement, l'ajout d'un contrôle interne d'amplification permet de vérifier les éventuelles inhibitions lors de la réaction. Tous les échantillons analysés (artificiellement ou non) ont donné des valeurs d'IPC identique (figure 6), ce qui confirme l'absence d'inhibiteur lors des réactions de PCR. Par conséquent, la méthode d'extraction développée est pertinente.

Les tests de détection *Brachyspira* ont été testés sur différents échantillons fournis par des laboratoires vétérinaires, présentant une suspicion d'infection de type spirochétose. Les échantillons sont traités dès leur arrivée, l'extraction des acides nucléiques est réalisée sur la plateforme Nuclisens® miniMAG^{™} Chaque échantillon est testé pur et dilué au 10^{ème} avec les trois couples d'amorce/sonde. Cependant aucune amplification pour les échantillons analysés n'a été observée. Pour confirmer l'absence d'amplification, un IPC a été rajouté au mix de PCR pour vérifier l'absence d'inhibiteurs de PCR.

Les trois espèces bactériennes ont été recherchées dans des échantillons de selles de porc contaminées artificiellement, afin de valider la méthode d'extraction et de détection. Les contaminations artificielles ont été réalisées avec des concentrations différentes. Pour chaque essai, une amplification a été observée sur les ADN extraits (figure 7), permettant de conclure que la méthode d'extraction et de détection est fiable sur des échantillons de types fèces. De plus, la technique d'extraction utilisée semble limiter la présence d'inhibiteurs de PCR puisque qu'un delta de valeurs de Ct d'environ 3,3 a été observé entre les échantillons purs et dilués au 1/10.

### Exemple 3 :

Différentes matrices à analyser prélevées sur porc ou volaille ont été envoyées par des vétérinaires, telles que des matières fécales, des écouvillons en pot stérile, des prélèvements intestinaux en pot stérile, des chiffonnettes en sachet stérile et de l'eau en bidon stérile.

### A - Prétraitement des échantillons

### Écouvillons

Les écouvillons (pool de 3 maximum) ont été mélangés sur vortex dans 2 ml de tampon de lyse, puis agités manuellement tout en prenant soin de ne pas faire mousser le mélange. Après incubation 10 min à Température Ambiante (TA) puis élimination des écouvillons, le mélange est centrifugé à 3000 g pendant 30 sec puis le surnageant conservé

### Matières Fécales et Prélèvements intestinaux

Une suspension de selles de 10 à 50 % dans 1 ml de PBS stérile a été effectuée, puis centrifugée 30 min à 3 000 rpm. Le surnageant obtenu est ensuite centrifugé 15 min à 10 000 t/min et l'opération répétée jusqu'à obtention d'un surnageant clair. Puis 300 µL de ce surnageant sont ajoutés à 2 ml de tampon de lyse Nuclisens bioMérieux, puis agité en évitant de faire mousser et incubé 10 min à température ambiante.

### Chiffonnettes

Les chiffonnettes ont été découpées de manière à obtenir un échantillon de 10 cm² au centre de chaque chiffonnette.

L'échantillon a ensuite été placé dans un sachet filtre stérile contenant 30 mL d'eau physiologique puis le mélange malaxé (si absorption totale, ajout 10 mL d'eau physiologique, et malaxer à nouveau). Enfin, 300 µL du surnageant filtré a été transféré dans 2 ml de tampon de lyse nuclisens bioMérieux, puis le tout a été agité en évitant de faire mousser et incubé 10min à température ambiante.

### Eaux

Une filtration d'IL d'eau est effectuée à l'aide de cassette de filtration tangentielle Sartorius Vivaflow 50. Un filtrat de 20 mL est récupéré. Afin de rincer la cassette, on fait passer 20 mL d'eau physiologique et on récupère 40 mL de filtrat dans un Falcon de 50 ml. A ce filtrat 10 ml de solution de PEG 6000 à 50% sont ajoutés puis le mélange est agité pendant 1h à 4°C (Table d'agitation rotatif). Après centrifugation à 11 000g pendant 30 min à 4°C, le culot est remis en suspension dans 1mL d'eau stérile chauffée à 56°C. Deux mL de tampon de lyse sont ajoutés et le mélange incubé 10 min à température ambiante.

### B - Extraction des acides nucléiques

Pour l'ensemble des matrices préparées ci-dessus, le procédé d'extraction des acides nucléiques est le même. La technique utilisée est la méthode d'extraction des acides nucléiques NucliSens de bioMérieux, mais le prétraitement sur le Kit d'extraction Qiagen et Invitrogen (selon les recommandations fournisseurs) a été également validé.

### C - PCR en temps réel

La réaction de PCR temps réel est réalisée avec des consommables, réactifs et appareil de chez Applied Biosystems, ces analyses ont également été réalisées sur d'autres types d'appareils (Stratagene MX3000...) et avec des réactifs de PCR en temps réel de fournisseurs différents (Invitrogen). Les résultats sont identiques.

### D - Programme PCR Temps réel

Un plan de plaque ou de barrette a été défini. Le programme présenté dans le tableau 10 pour le mélange réactionnel PCR temps réel avec IPC présenté dans le tableau 11 a été mis en oeuvre :

**Tableau 10 : Programme PCR Temps réel**

| Cycles | Temps | Température |
|---|---|---|
| 1 | 2 minutes | 50 °C |
| 1 | 10 minutes | 95 °C |
| 45 | 15 secondes | 95 °C |
| | 1 minute | 60 °C |

**Tableau 11: Mélange réactionnel PCR temps Réel avec IPC**

| Réactifs | Volume/Réaction en µl |
|---|---|
| H₂O | 3,25 |
| Master Mix | 12,5 |
| Master Mix IPC | 2,5 |
| DNA IPC | 0,5 |
| Amorce F (10µM) | 0,5 |
| Amorce R(10µM) | 0,5 |
| Amorce S (10µM) | 0,25 |

Les réactifs sont mis à décongeler sous une hotte dédiée à la préparation des Mix. Sous la hotte, on agite sur vortex et on centrifuge chaque réactif puis on les place sur la glace. On prépare ensuite le mélange réactionnel d'après le tableau 11.

On répartit 20 µL de Mix dans chaque puits, on dépose la plaque ou la barrette de tube sur un support.

Sous la hotte dédiée à la distribution des acides nucléiques, on ajoute 5µL d'échantillons par puits en respectant le plan de plaque ou de barrette de tube. On recouvre la plaque d'un film plastique ou de capuchon pour la barrette de tube. On centrifuge la plaque ou la barrette 10 secondes. Enfin, on met la plaque ou la barrette dans l'appareil pour démarrer la mesure.

### E - Résultats/interprétation

Une amplification sur les ADN extraits a également été observée pour un certain nombre d'échantillons (figure 7) permettant de conclure que le présent procédé est fiable sur les différentes matrices testées.

Ces essais ont ainsi permis de détecter sur 96 prélèvements terrains issus de porcs, les résultats positifs suivants :
*7 Brachyspira hyodisenteriae* (soit 7,30% des échantillons)
*15 Brachyspira pilosicoli* (soit 15,60% des échantillons)
*10 Brachyspira intermedia* (soit 10,40% des échantillons)

Concernant les 55 prélèvements issus de volailles (perdrix, dindes, gallus...), les résultats positifs sont les suivants :
*3 Brachyspira hyodysenterae* (soit 5,45% des échantillons)
12 *Brachyspira pilosicoli* (soit 21,80% des échantillons)
8 *Brachyspira intermedia* (soit 14,50% des échantillons)

L'efficacité et la sensibilité du test développé constituent une aide au diagnostic en permettant au vétérinaire de connaître et reconnaître les premiers signes cliniques des infections aux *Brachyspira spp.,* qui sont les plus souvent discrets.

Un des avantages intéressants de cette invention réside dans le gain de temps réalisé en utilisant la PCR en temps réel. En effet, les résultats peuvent être obtenus en 4 heures après réception des échantillons. Le temps d'analyse est un facteur très important pour les laboratoires vétérinaires et les vétérinaires conseils qui souhaitent intervenir le plus tôt possible sur les élevages.

Les méthodes de l'art antérieur, par PCR en point final couplée à la méthode culture ne permettent d'établir un diagnostic définitif que 5 à 7 jours après réception des échantillons. En outre, avec la présente invention, l'élimination des milieux spécifiques généralement onéreux, entraîne de fait une réduction des coûts liés au diagnostic.

Les échantillons soumis à l'essai sont des matrices contenant de nombreux inhibiteurs de PCR (matières organiques, sang, tissus...). Il était donc nécessaire de disposer d'une méthode d'extraction permettant de purifier efficacement les acides nucléiques extraits. La présente méthode d'extraction des acides nucléiques utilisés est simple, rapide et permet d'éliminer les inhibiteurs de PCR, l'ajout d'un contrôle d'amplification interne (IPC) permet de contrôler l'amplification.

Le test de détection de *"Brachyspira"* par PCR en temps réel développé présente toutes les caractéristiques exigées pour les analyses clients : rapidité, sensibilité, spécificité, pertinence et robustesse. La sensibilité du test est une caractéristique importante, celui-ci permet de détecter des échantillons ayant un niveau de contamination faible sachant qu'un très faible nombre de copies peuvent à elles seules représenter un risque pathogène. De plus, ce test permet non seulement de détecter mais aussi de différencier les différentes espèces de *Brachypira.*

En conclusion, le procédé selon la présente invention met en avant la possibilité d'utiliser une méthode de PCR en temps réel directement sur des prélèvements issus d'élevages.

Bien que l'invention ait été décrite en relation avec un mode de réalisation particulier, il est bien évident qu'elle n'y est nullement limitée.

## Revendications

1. Procédé de détection, d'identification et de quantification du gène *nox* (gène NADH oxidase) des espèces *Brachyspira hyodysenteriae, Brachyspira pilosicoli* et *Brachyspira intermedia*. issu d'un échantillon environnemental ou animal tel que des fèces de mammifères ou des fèces d'oiseaux, par réaction de polymérisation en chaîne (PCR), ladite réaction de polymérisation en chaîne (PCR) étant en temps réel, ledit procédé comprenant les étapes consistant à :
(i) analyser ledit échantillon susceptible de contenir des *Brachyspira* par PCR avec un milieu réactionnel comprenant une amorce sens apte à se lier spécifiquement au gène *nox*, une amorce anti-sens apte à se lier spécifiquement au gène *nox*, et une sonde d'hybridation moléculaire marquée fluorescente détectable qui se lie spécifiquement à l'amplicon résultant de la réaction PCR,
(ii) mesurer et quantifier, par l'intermédiaire d'un système optique d'un thermocycleur, la présence d'émission de fluorescence indiquant, de façon proportionnelle, la quantité d'amplicon à chaque cycle PCR, de manière à pouvoir détecter la présence de *Brachyspira* à partir dudit échantillon environnemental ou animal,
ledit procédé étant **caractérisé en ce que** :
- les amorces de *Brachyspira hyodysenteriae* comprennent les séquences suivantes : ATG TCC CAT ATA AAC ATC GAT GCC (amorce sens) et GTT GGT GGC GTA GTT AAA GAT CCT A (amorce anti-sens), la sonde de *Brachyspira hyodysenteriae* comprend la séquence suivante : CAC CTC TCA AAC TTT CAG GAC ;
- les amorces de *Brachyspira pilosicoli* comprennent les séquences suivantes : CAC TAG AAC AGT CTC CTA TAG CAT AAA CAT T (amorce sens) et A GAT TAT TTA GAG ACT TTA CCT AAT GGT (amorce anti-sens), la sonde de *Brachyspira pilosicoli* comprend la séquence suivante : CTCATCTTTAGAAGATTTCA ; et
- les amorces de *Brachyspira intermedia* comprennent les séquences suivantes : GTA TAG CAA TAG AGA ATG CAT GTA TAG CTT C (amorce sens) et GTA AAA ATA GTT TA T GAA GAA GAT ACT GGA CG (amorce anti-sens), la sonde de *Brachyspira intermedia* comprend la séquence suivante : CATGGTTGTTTTTAGAAGCA.

2. Procédé selon la revendication 1, dans lequel la sonde marquée détectable est une sonde d'hybridation moléculaire apte à accroître la spécificité de l'amplification comme les sondes TaqMan^{®}, TaqMan^{®} MGB^{™}.

3. Procédé selon l'une des revendications précédentes, dans lequel une molécule fluorescente ROX est ajoutée au mélange réactionnel, de manière à normaliser le signal complexe obtenu lors de la mesure de fluorescence.

4. Kit de détection, d'identification et de quantification du gène *nox* de l'espèce *Brachyspira* mis en oeuvre par le procédé de détection et de quantification selon l'une des revendications 1 à 3, comprenant les réactifs appropriés pour l'amplification du gène *nox* de l'espèce choisie parmi *Brachyspirahyodysenteriae, Brachyspira pilosicoli* et *Brachyspira intermedia*. issue d'un échantillon environnemental ou animal, en particulier issu de fèces de porcs et/ou de volailles, ledit kit comprenant un milieu réactionnel spécifique comportant :
(i) une amorce sens apte à se lier spécifiquement à la région 5'-3' du gène *nox,*
(ii) une amorce anti-sens apte à se lier spécifiquement à la région 3'-5' du gène *nox,*
(iii) une sonde d'hybridation moléculaire marquée fluorescente détectable qui se lie spécifiquement à l'amplicon (gène *nox*) résultant de la réaction PCR
(iv) des dNTPs et des polymérases présents en large excès dans le milieu réactionnel,
ledit kit étant **caractérisé en ce que** :
- les amorces de *Brachyspira hyodysenteriae* comprennent les séquences suivantes : ATG TCC CAT ATA AAC ATC GAT GCC (amorce sens) et GTT GGT GGC GTA GTT AAA GAT CCT A (amorce anti-sens), la sonde de *Brachyspira hyodysenteriae* comprend la séquence suivante : CACCTCTCAAACTTTCAGGAC ;
- les amorces de *Brachyspira pilosicoli* comprennent les séquences suivantes : CAC TAG AAC AGT CTC CTA TAG CAT AAA CAT T (amorce sens) et A GAT TAT TTA GAG ACT TTA CCT AAT GGT (amorce anti-sens), la sonde de *Brachyspira pilosicoli* comprend la séquence suivante : CTCATCTTTAGAAGATTTCA ; et
- les amorces de *Brachyspira intermedia* comprennent les séquences suivantes : G TAT AGC AAT AGA GAA TGC ATG TAT AGC TTC (amorce sens) et GTA AAA ATA GTT TA T GAA GAA GAT ACT GGA CG (amorce anti-sens), la sonde de *Brachyspira intermedia* comprend la séquence suivante : CATGGTTGTTTTTAGAAGCA.

5. Kit selon la revendication 4, **caractérisé en ce qu'**il comprend également :
(vi) un témoin interne positif (IPC),
(vii) un témoin positif
(viii) un témoin négatif

6. Kit selon l'une des revendications 4 ou 5, dans lequel la sonde marquée détectable est une sonde moléculaire apte à accroître la spécificité de l'amplification comme les sondes TaqMan^{®}, TaqMan^{®} MGB^{™}.

## Patentansprüche

1. Verfahren zum Nachweis, zur Identifizierung und zur Quantifizierung des Gens nox (Gen NADH Oxidase) der Arten *Brachyspira hyodysenteriae, Brachyspira pilosicoli* und *Brachyspira intermedia*, hervorgegangen aus einer Umwelt- oder Tierprobe wie Säugetier- oder Vogelfaeces durch Polymerase-Kettenreaktion (PCR), wobei die Polymerase-Kettenreaktion (PCR) in Echtzeit stattfindet, wobei das Verfahren die folgenden Schritte umfasst:
(i) Analyse der Probe, die *Brachyspira* enthalten könnte, durch PCR mit einem Reaktionsmedium, das einen Sense-Primer, der imstande ist, sich speziell an das Gen nox zu binden, einen Antisense-Primer der imstande ist, sich speziell an das Gen nox zu binden und eine nachweisbare fluoreszierend markierte molekulare Hybridationssonde, die sich speziell an das Amplikon im Ergebnis der PCR-Reaktion bindet, umfasst,
(ii) Messen und Quantifizieren anhand eines optischen Systems eines Thermocyclers des Vorhandenseins von Fluoreszenzemission, die für jeden PCR-Zyklus proportional die Menge Amplikon angibt, so dass das Vorhandensein von *Brachyspira* anhand der Umwelt- oder Tierprobe nachweisbar ist,
wobei das Verfahren **dadurch gekennzeichnet ist, dass**:
- die Primer von *Brachyspira hyodysenteriae* die folgenden Sequenzen umfassen: ATG TCC CAT ATA AAC ATC GAT GCC (Sense-Primer) und GTT GGT GGC GTA GTT AAA GAT CCT A (Antisense-Primer), wobei die Sonde von *Brachyspira hyodysenteriae* die folgende Sequenz umfasst: CAC CTC TCA AAC TTT CAG GAC,
- die Primer von *Brachyspira pilosicoli* die folgenden Sequenzen umfassen: CAC TAG AAC AGT CTC CTA TAG CAT AAA CAT T (Sense-Primer) und A GAT TAT TTA GAG ACT TTA CCT AAT GGT (Antisense-Primer), wobei die Sonde von *Brachyspira pilosicoli* die folgende Sequenz umfasst: CTCATCTTTAGAAGATTTCA, und
- die Primer von *Brachyspira intermedia* die folgenden Sequenzen umfassen: GTA TAG CAA TAG AGA ATG CAT GTA TAG CTT C (Sense-Primer) und GTA AAA ATA GTT TA T GAA GAA GAT ACT GGA CG (Antisense-Primer), wobei die Sonde von *Brachyspira intermedia* die folgende Sequenz umfasst: CATGGTTGTTTTTAGAAGCA.

2. Verfahren nach Anspruch 1, wobei die nachweisbar markierte Sonde eine molekulare Hybridationssonde ist, die imstande ist, die Amplifikationsspezifizität zu steigern, wie die Sonden TaqMan®, TaqMan® MGB™.

3. Verfahren nach einem der vorangehenden Ansprüche, wobei dem Reaktionsgemisch ein fluoreszierendes Molekül ROX derart hinzugefügt wird, dass das komplexe Signal, das man bei der Fluoreszenzmessung erhält, normalisiert wird.

4. Kit zum Nachweis, zur Identifizierung und zur Quantifizierung des Gens nox der Art *Brachyspira*, das mit dem Nachweis- und Quantifizierungsverfahren nach einem der Ansprüche 1 bis 3 umgesetzt wird, das Reagenzien umfasst, die für die Amplifikation des Gens nox der Art geeignet sind, die aus *Brachyspira hyodysenteriae*, *Brachyspira pilosicoli* und *Brachyspira intermedia* ausgewählt ist, hervorgegangen aus einer Umwelt- oder Tierprobe, insbesondere aus Schweine- und/oder Geflügelfaeces, wobei das Kit ein spezifisches Reaktionsmedium umfasst, das aufweist:
(i) einen Sense-Primer, der imstande ist, sich speziell an die Region 5'-3' des Gens nox zu binden,
(ii) einen Antisense-Primer, der imstande ist, sich speziell an die Region 3'-5' des Gens nox zu binden,
(iii) eine nachweisbare fluoreszierend markierte molekulare Hybridationssonde, die sich im Ergebnis der PCR-Reaktion speziell an das Amplikon (Gen nox) bindet"
(iv) dNTPs und Polymerasen in erheblichem Überschuss im Reaktionsmedium,
wobei das Kit **dadurch gekennzeichnet ist, dass**:
- die Primer von *Brachyspira hyodysenteriae* die folgenden Sequenzen umfassen: ATG TCC CAT ATA AAC ATC GAT GCC (Sense-Primer) und GTT GGT GGC GTA GTT AAA GAT CCT A (Antisense-Primer), wobei die Sonde von *Brachyspira hyodysenteriae* die folgende Sequenz umfasst: CACCTCTCAAACTTTCAGGAC,
- die Primer von *Brachyspira pilosicoli* die folgenden Sequenzen umfassen: CAC TAG AAC AGT CTC CTA TAG CAT AAA CAT T (Sense-Primer) und A GAT TAT TTA GAG ACT TTA CCT AAT GGT (Antisense-Primer), wobei die Sonde von *Brachyspira pilosicoli* die folgende Sequenz umfasst: CTCATCTTTAGAAGATTTCA, und
- die Primer von *Brachyspira intermedia* die folgenden Sequenzen umfassen: G TAT AGC AAT AGA GAA TGC ATG TAT AGC TTC (Sense-Primer) und GTA AAA ATA GTT TAT GAA GAA GAT ACT GGA CG (Antisense-Primer), wobei die Sonde von *Brachyspira intermedia* die folgende Sequenz umfasst: CATGGTTGTTTTTAGAAGCA.

5. Kit nach Anspruch 4, **dadurch gekennzeichnet, dass** es ebenfalls umfasst:
(vi) eine positive interne Kontrolle (IPC),
(vii) eine positive Kontrolle,
(viii) eine negative Kontrolle.

6. Kit nach einem der Ansprüche 4 oder 5, wobei die nachweisbar markierte Sonde eine molekulare Hybridationssonde ist, die imstande ist, die Amplifikationsspezifizität zu steigern, wie die Sonden TaqMan®, TaqMan® MGB™.

## Claims

1. A method for detecting, identifying and quantifying the gene *nox* (NADH oxidase gene) of the species *Brachyspira hyodysenteriae, Brachyspira pilosicoli* and *Brachyspira intermedia* from an environmental or animal sample such as mammal faeces or bird faeces, by a polymerization chain reaction (PCR), said polymerization chain reaction (PCR) occurring in real time, said method comprising the steps:
(i) analyzing said sample which may contain *Brachyspira* by PCR with a reaction medium comprising a sense primer able of specifically binding to the gene *nox,* an anti-sense primer able of specifically binding to the gene *nox,* and a detectable fluorescence-marked molecular hybridization probe which specifically binds to the amplicon resulting from the PCR reaction,
(ii) measuring and quantifying, via an optical system of a thermocycler, the presence of fluorescence emission of proportionally indicating the amount of amplicon at each other PCR cycle, so as to be able to detect the presence of *Brachyspira* from said environmental or animal sample,
said method being **characterized in that**:
- the primers of *Brachyspira hyodysenteriae* comprise the following sequences: ATG TCC CAT ATA AAC ATC GAT GCC (sense primer) and GTT GGT GGC GTA GTT AAA GAT CCT A (anti-sense primer), the probe of *Brachyspira hyodysenteriae* comprises the following sequence: CAC CTC TCA AAC TTT CAG GAC;
- the primers of *Brachyspira pilosicoli* comprise the following sequences: CAC TAG AAC AGT CTC CTA TAG CAT AAA CAT T (sense primer) and A GAT TAT TTA GAG ACT TTA CCT AAT GGT (anti-sense primer), the probe of *Brachyspira pilosicoli* comprises the following sequence: CTCATCTTTAGAAGATTTCA; and
- the primers of *Brachyspira intermedia* comprise the following sequences: GTA TAG CAA TAG AGA ATG CAT GTA TAG CTT C (sense primer) and GTA AAA ATA GTT TA T GAA GAA GAT ACT GGA CG (anti-sense primer), the probe of *Brachyspira intermedia* comprises the following sequence: CATGGTTGTTTTTAGAAGCA

2. The method according to claim 1, wherein the probe marked as detectable is a molecular hybridization probe able to increase the specificity of the amplification like the TaqMan®, TaqMan® MGB™ probes.

3. The method according to one of the preceding claims, wherein a fluorescent molecule ROX is added to the reaction medium, so as to normalize the complex signal obtained during the fluorescence measurement.

4. A kit for detecting, identifying and quantifying the gene nox of the *Brachyspira* species applied by the detection and quantification method according to one of claims 1 to 3, comprising the reagents suitable for amplifying the gene nox of the species selected from *Brachyspira hyodysenteriae, Brachyspira pilosicoli* and *Brachyspira intermedia,* stemming from an environmental or animal sample, in particular from pig and/or poultry faeces, said kit comprising a specific reaction medium including:
(i) a sense primer able to specifically bind to the 5'-3' region of the gene nox,
(ii) an antisense primer able to specifically bind to the 3'-5' region of the gene nox,
(iii) a detectable fluorescence-marked molecular hybridization probe which specifically binds to the amplicon (gene nox) resulting from the PCR reaction,
(iv) dNTPs and polymerases present in a large excess in the reaction medium,
that said kit being **characterized in that**:
- the primers of *Brachyspira hyodysenteriae* comprise the following sequences: ATG TCC CAT ATA AAC ATC GAT GCC (sense primer) and GTT GGT GGC GTA GTT AAA GAT CCT A (anti-sense primer), the probe of *Brachyspira hyodysenteriae* comprises the following sequence: CACCTCTCA AACTTTCAGGAC;
- the primers of *Brachyspira pilosicoli* comprise the following sequences: CAC TAG AAC AGT CTC CTA TAG CAT AAA CAT T (sense primer) and A GAT TAT TTA GAG ACT TTA CCT AAT GGT (anti-sense primer), the probe of *Brachyspira pilosicoli* comprises the following sequence: CTCATCTTTAGAAGATTTCA; and
- the primers of *Brachyspira intermedia* comprise the following sequences: G TAT AGC AAT AGA GAA TGC ATG TAT AGC TTC (sense primer) and GTA AAA ATA GTT TAT GAA GAA GAT ACT GGA CG (anti-sense primer), the probe of *Brachyspira intermedia* comprises the following sequence: CATGGTTGTTTTTAGAAGCA.

5. The kit according to claim 4, **characterized in that** it also comprises:
(vi) a positive internal control (PIC),
(vii) a positive control,
(viii) a negative control.

6. The kit according to one of claims 4 or 5, wherein the detectable marked probe is a molecular probe able to increase the specificity of the amplification like the TaqMan®, TaqMan® MGB™ probes.
